# EUROPEAN PATENT APPLICATION

(11) **EP 4 052 792 A1**
(43) Date of publication of application: **07.09.2022**
(21) Application number: 21161107.4
(22) Date of filing: 05.03.2021
(51) Int. Cl.: B01L 3/00, G01N 21/77, G01N 21/84, G01N 21/78, G01N 33/558

(54) **DEVICE AND METHOD FOR ELECTRONICALLY READING AND TRANSMITTING THE RESULTS OF A BIOCHEMICAL TEST**

(71) Applicant: MediqC19 B.V., 2913 LZ Nieuwerkerk aan den IJssel (NL)
(72) Inventor: DE WIT, Jan Hendrik, 2913 LZ Nieuwerkerk aan den IJssel (NL); VISSER, Robbert Jan Herman, 2913 LZ Nieuwerkerk aan den IJssel (NL)
(74) Representative: Hoyng Rokh Monegier B.V.

(57) **Abstract**

The invention relates to a testing device for detecting the colour-change result of a biochemical test arranged to detect the presence of a target substance in a liquid sample. The testing device comprises a receptacle for receiving the biochemical test; one or more sensor systems for reading the output of the biochemical test; a microcontroller connected to the one or more sensor systems for processing data from the one or more sensors; wherein the testing device is characterized by: the one or more sensor systems comprising a light source and a photodetector; wherein the light source is arranged to irradiate with visible light an area of the biochemical test that changes colour, wherein the visible light is of a different colour than the colour to which the area can change; and wherein the photodetector is arranged to measure an optical property of the area that is irradiated.

## Description

### TECHNICAL FIELD

This disclosure generally relates to the field of lateral flow tests for determining the presence of a target substance in a liquid sample. In particular, the invention relates to a testing device and method for electronically reading and transmitting the results of such a test.

### BACKGROUND

Polymerase chain reaction (PCR) tests test for a target substance. PCR tests are widespread and well known. The PCR test is a test to show whether you are currently infected with the coronavirus (SARS-CoV-2). A swab, such as a throat and nasal swab taken by a doctor, forms the basis of the PCR test. The collected material can be analysed in a laboratory. This results in a negative or positive result. This method is quite involved and requires heating and cooling the sample to obtain larger quantities of DNA.

Lateral flow tests, also known as lateral flow immunochromatographic assays or rapid tests, are simple devices intended to detect the presence of a target substance in a liquid sample without the need for specialized and costly equipment. These tests can be performed by a medical professional, or at home. The interpretation of these tests can be counterintuitive, and when performing these tests at home there is currently no direct way of authenticating a result, nor is there currently a good way to submit these results in a direct and secure way to a medical database or medical professional for a follow-up.

It is therefore an object of the invention to solve the abovementioned problems relating to biochemical tests.

### DESCRIPTION OF THE INVENTION

To address the above discussed drawbacks of the prior art, the present invention provides a method according to the appended claims and embodiments disclosed herein. The invention furthermore provides an apparatus comprising a housing with receptacle for a lateral flow test and sensor systems for reading out the results. The invention also provides an assembly and/or kit-of-parts of a testing housing and one or more lateral flow tests, wherein the testing housing is suitable to receive and read out the test results of multiple lateral flow tests, wherein the housing has an opening for inserting a lateral flow test into a receptacle in the housing, wherein the opening, lateral flow test and receptacle, as well as the sensor systems in the housing have a predetermined matching size. In particular the sensor systems are adapted to read-out respective test surface areas on the lateral flow test, when the lateral flow test is inserted.

### BRIEF DESCRIPTION OF DRAWINGS

Embodiments will now be described, by way of example only, with reference to the accompanying schematic drawings in which corresponding reference symbols indicate corresponding parts, and in which:
- FIGS. 1A-D: illustrate how to obtain a processed specimen from a swab specimen.
- FIG. 2: shows how the processed specimen is brought in contact with a device for detecting the presence of a target substance in a liquid sample.
- FIG. 3: shows an exemplary biochemical test which can be used in the present invention.
- FIG. 4: shows the possible results of a biochemical test which can be comprised in the device for detecting the presence of a target substance.
- FIG. 5: shows an exemplary arrangement of sensor placement in the device for detecting the presence of a target substance in a liquid sample.
- FIG. 6: schematically shows an exemplary embodiment of the testing device for detecting the presence of a target substance in a liquid sample connected with a second device.
- FIGS. 7A-B: show embodiments of a testing device for a single lateral flow test.
- FIGS. 8A-B: show a testing device for a single lateral flow test.
- FIGS. 9A-B: show embodiments of a testing device with two receptacles for two lateral flow tests.

The figures are intended for illustrative purposes only, and do not serve as restriction of the scope or the protection as laid down by the claims. Further any feature is disclosed in relation to an embodiment can be combined with features of the other embodiments, unless specifically indicated that they can not be combined.

### DESCRIPTION OF EMBODIMENTS

Hereinafter, certain embodiments will be described in further detail. It should be appreciated, however, that these embodiments may not be construed as limiting the scope of protection for the present disclosure. Although the invention is described in a non-limiting manner with reference to a lateral flow test, the invention can be used with and/or applied to any biochemical test.

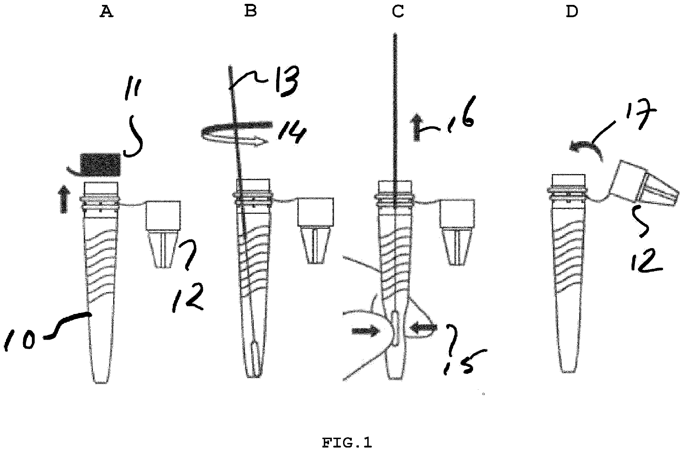

FIGS. 1A-D illustrate how to obtain a processed specimen from a swab specimen.

The swab specimen is obtained from a user, for example a (potential) patient. The swab specimen can comprise a sample of for example a bodily fluid that may or may not comprise a target substance for which one would like to test. For example, in the case of COVID-19 proteins being the target substance, the swab specimen can be obtained by insert the swab into a nostril of a patient, gently rubbing and rolling the swab and leaving it in place for several seconds to absorb the specimen, then slowly removing the swab while rotating it.

In FIG. 1A, the lid 12 is taken off a process tube 10. The process tube 10 comprises a processing fluid. The processing fluid reacts with the specimen. The processing tube furthermore can comprise a dripper 12. The dripper 12 is preferably separate from the lid. In another embodiment, the lid can be a dripper with a removable seal.

In FIG. 1B the swab 13 comprising the swab specimen is put into the tube 10. The swab can be rotated 14 in order to better release the swab specimen into the processing fluid.

In FIG. 1C the bottom of the tube is squeezed 15 while the sample is inside. This ensures that the swab specimen is better released into the processing fluid. The processing tube 10 is therefore preferably made from a flexible material, for example transparent plastic.

In FIG. ID the dripper 12 is placed 17 on the processing tube 10. In this way spillage of the processed fluid is limited. The processing tube 10 can be shaken before or after the dripper is placed on, to make sure that the processed specimen is mixed well. This can be done by first placing the lid on the processing tube, or in the case that the dripper does not release processing fluid when shaken, this can be done while the dripper 12 is on the processing tube 10.

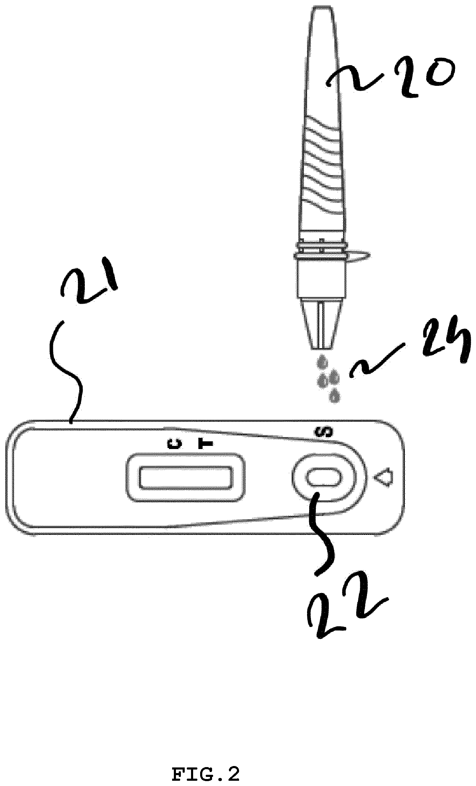

FIG. 2 shows how the processed specimen 24 from the processing tub 10 is brought in contact with a device 21 for detecting the presence of a target substance in a liquid sample. A waiting time, e.g. 2-3 minutes, can be required.

The device 21 can comprise an opening 22, where the processed specimen 24 can be inputted into the device. The opening 22 has the analyte input. The analyte input can for example be a sample well. For example, drops of the processed specimen 24 can be dropped into the sample well from the processing tube 10. The processed specimen 24, after having entered the device via the analyte input is then subsequently tested.

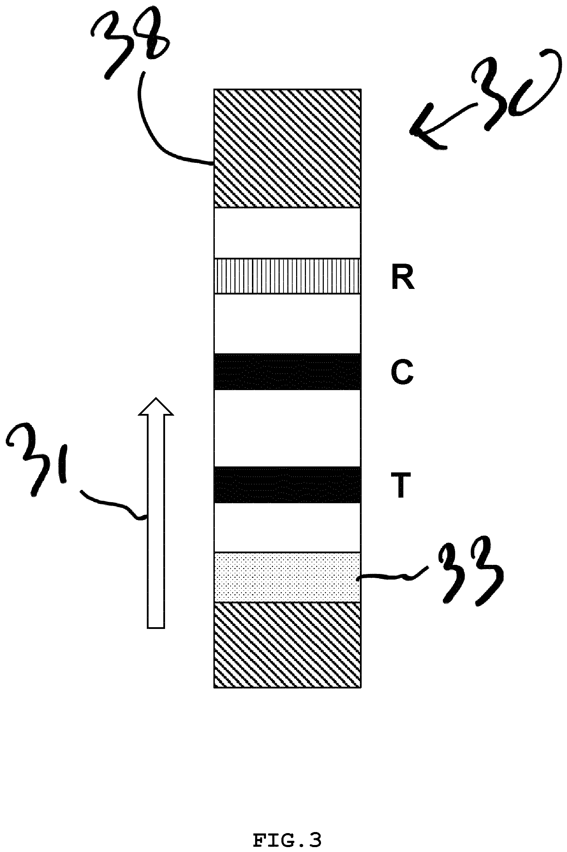

FIG. 3 shows an exemplary lateral flow test that can be used in the present invention. The lateral flow test of the present embodiment is an example of a biochemical test, also called a lateral flow (immunochromatographic) assay or rapid test. The analyte, e.g. the processed specimen, is input on a sample pad of the test strip 30.

The lateral flow test runs the liquid sample along the surface of the pad 30, shown with the arrow 31. The pad 30 comprises reactive molecules that show a visual positive or negative result. For example, the visual positive result is the formation of a red line in a particular area of the lateral flow test. Pads can be formed as a series of capillary beds, for example pieces of porous paper, a microstructured polymer, and/or sintered polymer. Each of these pads has the capacity to transport fluid (e.g., urine, blood, saliva, or processed specimen) spontaneously.

After the liquid specimen has entered the device 10 via the opening 22, the sample comes into contact with the sample pad 33. The sample pad acts as a sponge and can hold an excess of sample fluid. Once appropriately soaked, the fluid flows in the direction of the arrow 31. An optional second pad, a so-called conjugate pad can store freeze dried bio-active particles called conjugates in for example a salt-sugar matrix. The conjugate pad contains all the reagents required for an optimized chemical reaction between the target molecule (for example an antigen) and its chemical partner (for example an antibody). The conjugate particles thus mark target particles as they pass through the pad and continue across to the test and control lines (C,G,T).

In a different embodiment, the conjugate particles are for example present in the processing tube, and are allowed to react with the target particles of the swab specimen in the processing tube before the liquid specimen is inputted into the device.

The test line T shows a signal, often a colour such as red as in for example COVID-19 tests. The control line C contains affinity ligands which show whether the sample has flowed through and the bio-molecules used as the conjugate particles are active. The signal for the control line C can be the same as the signal for the test line T, i.e. a red line. After passing these reaction zones, the fluid enters the final porous material, the wick 38, that simply acts as a waste container.

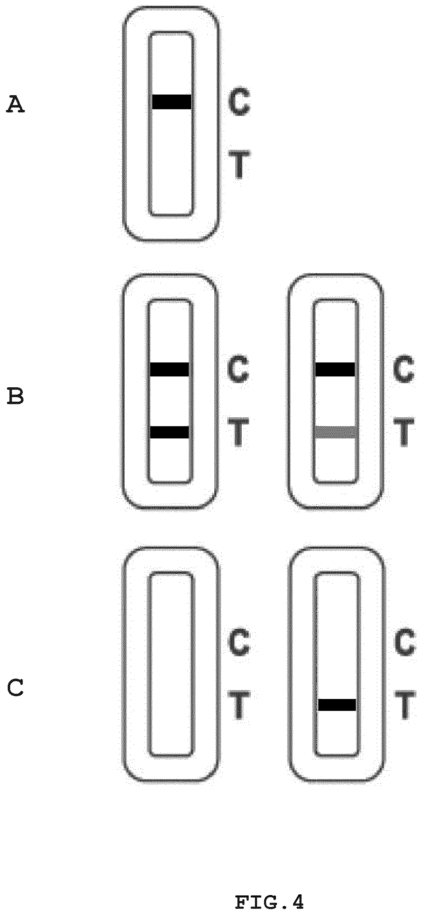

FIG. 4 show the possible results of a lateral flow test for COVID19, which can be comprised in the device for detecting the presence of a target substance. In FIG. 4 row A, what is shown is a negative result. The control line C indicates that the conjugate particles are active, but the test line T shows that there have been no target particles found in the analyte. In FIG. 4 row B, what is shown is a positive result. The control line C indicates that the conjugate particles are active, and the test line shows that there are target particles present in the analyte. Even a faint test result indicates that target particles are present in the sample. In FIG. 4 row C, what is shown are inconclusive results. In both cases the control line C indicates that the conjugate particles are inactive.

The plurality of results possible from a single test makes the test result difficult to interpret by a lay person by these visual cues only. Furthermore, it is beneficial to digitize the results immediately, such that less human errors can be made in interpreting or transcribing the results. The results of the test can be more easily shared with third parties, for example a medical professional, a patient's electronic dossier, a database or other parties. There is furthermore a need for a cheap and simple arrangement which can be easily manufactured and provide these benefits.

As shown in Figure 3, a third line forms a reference area R, which can be used together with the test line T and control line C by a sensor arrangement of the present invention to automatically measure and digitize the test result of the device. The reference area R does not comprise particles that react with the flowing liquid sample and thus does not show the same signal as either the test line T, or the control line C. For example, while the control line C and test line T may colour red because of the target particle's presence in the liquid sample, the reference area R remains in the same colour as the rest of the pad, for example white.

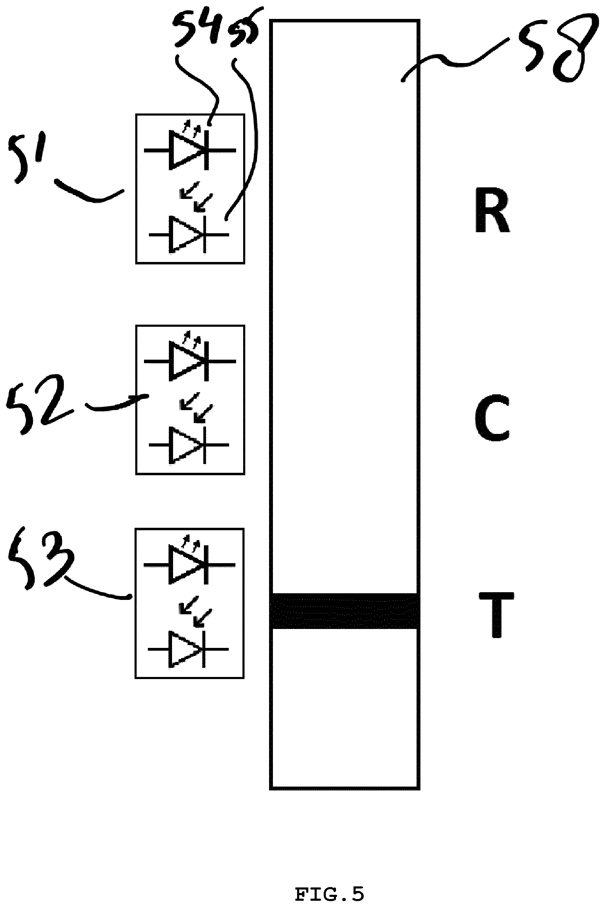

FIG. 5 shows an exemplary arrangement of sensor systems in the device for detecting the presence of a target substance in a liquid sample.

In the housing of the testing device a receptacle is formed for pad 30. That receptacle is preferred sized to correspond with a predetermined pad 30. This allows to position the pad 30 with respect to the sensor system. In the vicinity of the test area T, the control area C and the reference area R a sensor system is arranged. Each sensor system 51-53 according to a preferred embodiment comprises an LED 54 and an optoelectronics reader 55 as light source 54 and photodetector 55 respectively. Any light emitter can be used. The optoelectronics reader can be an infrared sensor. The LED irradiates or directs light on the particular area beneath which it is placed. The light is either absorbed by the surface of the area or reflected of the surface of the area.

In the below reference is made to colours. It will be clear to the skilled man that any colour can be used. Colours refer to a wavelength domain of visible light. The wavelength domain can be 20nm, 30nm or 50nm wide. Red refers to 664-680 nm, green refers to 534-545 nm; blue refer to 420-440 nm. Broader ranges are possible. Other preferred ranges are 600-800nm, 580-750nm and 620-700nm.

For example, the LEDs shine a blue light on the test area T, the control area C and the reference area R. The test area T is coloured red. The control area C is not coloured, and thus has the same colour as the rest of the pad. The reference area R does not colour, an thus also has the same colour as the rest of the pad. Thus, the control area C and the reference area R are for example coloured white. Because the blue LED light and the red signal value are of different colour, the blue LED light is absorbed by the red signal area. However, the blue LED light is reflected by the white control area C and the reference area R. Any other visible colour can be used, as long as the LED light colour and the signal colour in the test area T and the control area C are different. The colour of the signal in the test area T and control area C can be different from each other.

The blue light is absorbed by the red line in the test area T, while the blue light is reflected by the white pad in the control area C and the reference area R. The optoelectronics reader thus receives a different intensity value in the test area T, than in the control area C and the reference area R. For example, the intensity in the test area T is lower than the intensity in both the control area C and the reference area R. The intensity values in the control area C and the reference area R are approximately the same. Thus, based on these measurements, it can be inferred that the test area T shows that apparently target particles have been found in the analyte. However, it can also be inferred that the control area C indicates that no conjugate particles are active. Hence, it can be concluded based on this measurement that the test result is inconclusive.

A single sensor system could be used, which measures the test area T, control area C and reference area R in sequence. The sensor system can for example be movably placed in the device in this case. However, it is preferred that all areas are measured at the same time, to cope with changing conditions in the lighting of for example the environment in which the test is performed.

In principle, a pre-determined reference value R can also be used to compare with the sensor output for the control area C and test area T. However, the use of reference area R is preferred, since it serves to cope with the changes in environment conditions, for example the amount of ambient light reaching the pad and/or sensor system. Furthermore, the test area T and control area C can be quite small, and are normally bordered by white-coloured pad areas. Thus, a calibration measurement is important.

The photodetector 55 can provide an analogue or digital output signal. The output signal can be a digitally sampled output signal.

In an embodiment the unprocessed measurement data can be sent to a processor for processing the measurement data of the sensors. In other embodiments the photodetector 55 has a processor. The processor allows to compare the output signal of the photodetection to a predetermined threshold, the threshold being (amongst others) dependent on the calibration measurement. The processed output signal can be a one bit signal: yes or no.

In embodiments the processed or unprocessed output signal of the photosensors is sent to a central processor (not shown) connected to the preferably each sensor system 51-53. The central processor, in some embodiments formed by a simple combination of transistors, can derive a result from the combined measurement data; for example a positive, negative or inconclusive result.

In embodiments, the central processor can also prepare the raw data of the sensors so that these can be sent to another device for further processing and interpretation. For example, the data can be sequenced appropriately before it is sent to the other device.

Note that if a later flow test for example does not have a control area then the sensor reading for that area can be ignored, or that sensor system can be omitted from the device.

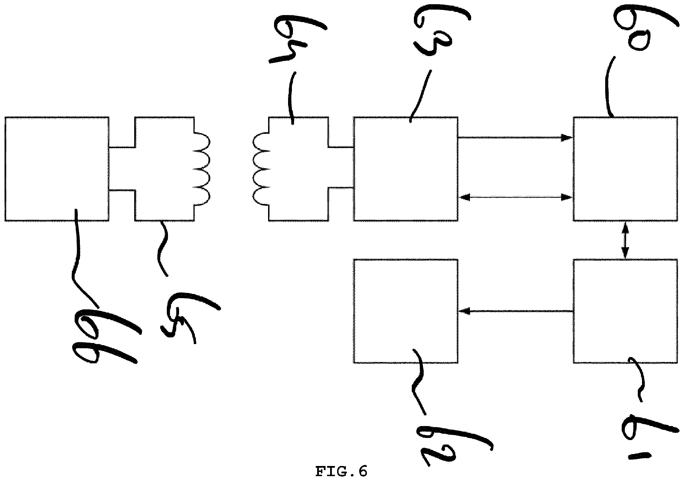

FIG. 6 schematically shows an exemplary embodiment of the testing device for detecting the presence of a target substance in a liquid sample connected with a second device 66.

The second device 66 can for example be a smart phone, or any other device suitable for communicating with the testing device. The testing device comprises one or more sensor systems 61, a microcontroller 60 and a transmitter or transceiver 63. The sensor system 61 can read out one or more properties, e.g. optical, of a lateral flow test 62.

Sensors read the biochemical test, for example as described above. The sensor readings are converted by an Analogue-to-Digital convertor to a digital signal, and via an Input/Output connection sent to the microcontroller 60, which processes the sensor data by use of its processor. In this embodiment, the microcontroller stands in connection with the transmitter or transceiver 63, for example via an I²C bus. However, the testing device can also comprise a display system or a different kind of communication system to display or communicate the test result to a user of the testing device. In embodiments the transceiver 63 can comprise coils or capacitors for power storage, preferably coils that can be powered via connection 64

In this embodiment, the second device comprises a receiver or transceiver and is arranged to receive and interpret the processed data from the processor. The connection 64,65 between the testing device and the second device can be made through any suitable connection method, for example via Wi-Fi, Bluetooth, NFC or similar connection method.

The transmitter 63 can be arranged as a transponder with respect to an initiator device that provides an electromagnetic field which the transponder modulates, preferably wherein the testing device draws all its operating power from the electromagnetic field provided by the initiator device 66, more preferably wherein the transmitter is arranged to use the Near-Field Communication (NFC) communication protocol. The initiator device is in this case the second device, for example a smart phone that can communicate via NFC.

In a preferred embodiment, the transmitter or transceiver 63 is an NFC transceiver and power harvester. In this way, the testing device does not need batteries and can be powered by the second device, which also comprises an NFC transceiver. The testing device then comprises an NFC RX coil, and the second device comprises an NFC TX coil.

Since most modern smartphones can communicate via NFC, this connection method can be used by a user of the testing device to the user's smartphone. Furthermore, advantageously since the testing device can be powered via the NFC connection, no battery is needed. This makes the testing device simpler and cheaper to manufacture.

Once the second device has received the processed data, appropriate actions can be taken based on the processed data. The second device can relay the data to a physician.

The communication between the testing device and the second device can also be performed via RFID. In an embodiment, a unique code of the testing device is embedded in the microprocessor 60 or in the sensor system 61 or in a (not shown) memory. The unique code can be transmitted to the second device via an RFID chip in the testing device. This allows verifying that a particular, uniquely identified, testing device is/was used for the test. E.g. a doctor provides the testing device with unique code 1234 to a user. Only when that unique code (or a derived code therefrom) is received from that user, via the second device, will this lead to an authentication of the result of the test by that doctor.

The testing device can be utilized as follows. The user obtains the testing device comprising one or more lateral flow tests. The user can use an application on the user's phone, or on any other device that the user plans to use with the testing device. Via the application, a unique code can be entered that identifies the testing device and/or the biochemical test. The unique code can be present on an RFID chip and submitted to the application via the second device. The unique code can also be visible on the outside of the testing device and manually entered in the application. The unique code can also be present in a memory on the testing device and transmitted to the second device, for example via NFC. A unique code is in some cases not necessary in order to use the testing device, but it can give extra security or serve as a way to couple the user's identity to the test result.

The user can activate the test measurement either in the application on the second device, or by controlling the power on the testing device itself. Preferably the testing device draws all its operating power from the second device. By using the application on the second device to active the test measurement, the second device can send electromagnetic energy to the testing device. In a first step, the central processor communicates the status of the testing device, e.g. ready for test or 'already used'. The user can either choose when the one or more sensor systems in the testing device measure the test result, or the measurement can be performed after a pre-determined time.

The pre-determined time can be manually set by the user in the application, or can be pre-determined based on the type of biochemical test that is performed. For example, for certain COVID-19 tests, the test result should be read after 15 minutes and not later than 20 minutes.

The application obtains the processed data and it is thus determined whether the test was performed successful or unsuccessful, and whether the target substance was present or not. For example, the application displays this information to the user via the second device.

The application can be coupled with a medical database where the result can be automatically entered. The user might receive a push notification in the case of a positive result, for example a push notification a couple of days after the test was taken to check whether the user went to see a health professional. The application can couple a health professional to the user via its interface to give medical advice.

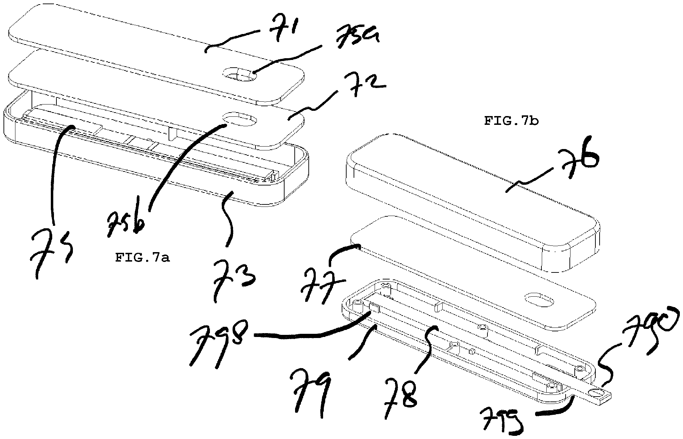

The user performs the biochemical test by bringing the analyte in contact with the biochemical test. The biochemical test can be affixed within the testing device as shown in Figure 7a, or can later be brought into contact with the testing device, for example by placing the biochemical test into the testing device, such as the embodiment of Figure 7b.

The lateral flow test 74 can be unremovably fixed to a housing part 73 of the testing device. Housing part 73 has a receptacle in which the test 74 is positioned. Housing part 73 together with cover 71 form a house in which the test 74 is received. A circuit board 72 is also received in the housing. The circuit board 72 can be embodied similar to figure 6. Sample opening 75a is, when assembled, aligned with opening 75b in the circuit board, and allows some specimen to be put on pad 74. Circuit board 72 has, on a bottom side, sensor systems that allow reading out the results of the bars. In the Figure 7a embodiment the microprocessor present on the circuit board can have a unique code for identifying the testing device. A bar code can be present on the outside of the housing to provide a unique code.

Figure 7b shows a similar housing of a testing device formed by housing part 79 and cover 76. Housing part 79 has a receptacle in which a lateral flow test 78 can be positioned. A tip of the lateral flow test will extend from the assembled housing on the right hand side. The lateral flow test 78 can be inserted through the opening formed between cover and housing part so that it is positioned in a receiving space within the housing. In the shown embodiment of Figure 7b, specimen of the fluid to be tested is applied to the test pad of the lateral flow test 78 before inserting the lateral flow test into the side opening of the housing. In other embodiments the test device having a replaceable lateral flow test can allow the application of specimen only after inserting the lateral flow test.

In embodiments the sensor systems are arranged to ascertain the correct positioning of the lateral flow test 78 in the housing. E.g. measurements can be performed to ascertain that the control area of the test is positioned in the vicinity or irradiated area of one of the sensor systems. When the lateral flow test 78 is positioned correctly in the housing, sensor systems on the circuity board 77, which can be same circuit board as circuit board 72, can irradiate and read out the test results by positioning the respective areas in the vicinity of the sensor systems.

In the case that the biochemical test is not an inherent part of the testing device, the testing device can be reused for reading the results of a different biochemical test.

The invention also relates to a kit of parts of a testing device and lateral flow test. The testing device can have any of the features disclosed herein. However the sensor system can be electrical or without a light source. The lateral flow test 78 comprises a sample pad 790 that, when the lateral flow test 78 is inserted in the opening 799 of the housing 76,79, extends from that opening. That way contact of the possibly infected fluid with the testing device is prevented.

To guide insertion of the lateral flow test 78 into the opening 799 and housing 76,79, guide blocks 798 are provided. The receptacle for the lateral flow test 78 is formed between the guide blocks 798.

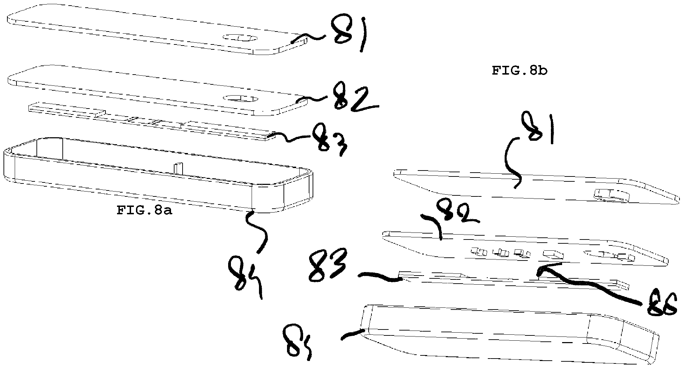

Figure 8a and 8b show two different exploded views of the same embodiment of a testing device according to an embodiment of the invention. A testing device comprises a housing part 84 and a cover 81. They can be connected to form a closed of housing. Within the housing, in housing part 84, a receptacle is formed in which a lateral flow test 83 is affixed. The receptacle and lateral flow test 83 are dimensioned such that a sample receiving area of the lateral flow test 83 is aligned with an opening in the circuit board 82 and an opening in the cover 81. This allows positioning specimen to be tested on the sample receiving area.

A removable protection, e.g. a protective foil, can be present to protect the opening and sample area from undesired use. The protection can be removed shortly before depositing the specimen. The protection can be coupled to the microprocessor. If the protection is removed more than a certain time period, e.g. 5 minutes, before the specimen is received, the microprocessor can e.g. be blocked from further use. In embodiments, upon activation of microprocessor, e.g. via NFC, the microprocessor can test the presence of the protection and, if missing, can provide an error code.

The circuit board 82 has sensor systems 88 provided on the side of the circuit board directed at the receptacle, where the lateral flow test is positioned. In Figure 8b, three sensor systems, each including a light and photodetector, are present. In embodiments a single sensor system comprising only a photodetector can be present. In other embodiments a sensor system using a sensing technique different from optics can be used, e.g. electrical / resistance measurements.

The sensor system is arranged to detect the result of the lateral flow test, in particular of one or more test result areas on the lateral flow test strip. The sensor system and lateral flow test are adapted to each other to allow reliable read-out of the test results.

On the circuit board 82 a microprocessor coupled to the sensor systems can be present. Any of the features of Figure 6 can present on the circuit board 82.

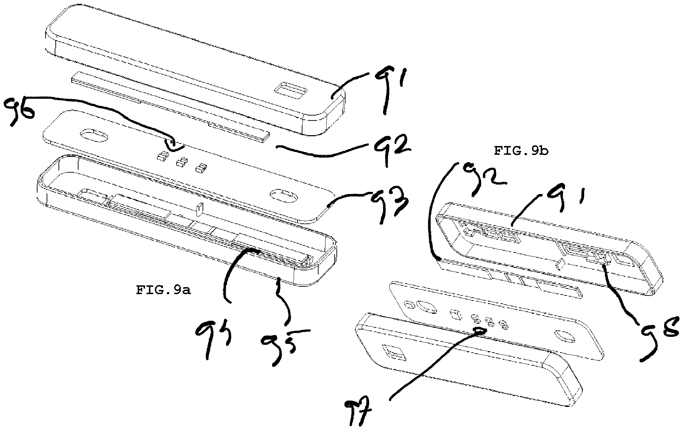

In Figures 9a and 9b an embodiment of a testing device having two receptacles for two lateral flow tests is disclosed. Receptacle 98 in cover 91 is visible in Fig 9b. Cover 91 and housing part 95 form a housing that receives a circuit board 93 and two lateral flow tests 92,94. The circuit board is sandwiched between the receptacles for the two lateral flow tests 92,94. The circuit board 93 has on a side facing lateral flow test 92 sensor systems 96 and has on a side facing lateral flow test 94 sensor systems 97.

Two sample receiving openings are formed in the housing, one in cover 91 and one opening in housing part 95. Circuit board 93 has two openings.

By providing the circuit board with at least one opening for allowing the specimen to be positioned on a testing area of the lateral flow test, the specimen can be positioned on a side of the lateral flow test facing the circuit board, which increases the reliability of the read-out and thereby of the test.

Two or more of the above embodiments may be combined in any appropriate manner. In embodiments the testing device is provided in combination with container holding sanitizing material. In embodiments the microprocessor will block further usage after detecting a positive COVID-19 result.

## Claims

1. A testing device for detecting a colour-change result of a lateral flow test arranged to detect the presence of a target substance in a liquid sample, the testing device comprising:
one or more receptacles for receiving one or more lateral flow tests;
one or more sensor systems for reading a colour-change result of the one or more lateral flow tests;
a microcontroller connected to the one or more sensor systems for processing data from the one or more sensor systems;
wherein the one or more sensor systems comprise one or more light sources and one or more photodetectors;
wherein the light source is arranged to irradiate into the receptacle onto an area of the lateral flow test that changes colour;
wherein the photodetector is arranged to measure an optical property of the area that is irradiated; and
wherein the light source, the colour-change result of the lateral flow test and the photodetector configured to cooperate to allow measuring the colour-change result.

2. The testing device of claim 1, wherein the light source is arranged to irradiate light having a predetermined wavelength range, preferably visible light, wherein the irradiated light is of a different wavelength, preferably colour, than the wavelength range, preferably colour, to which the lateral flow test can change, wherein preferably the photodetector is arranged to measure light in the range of wavelengths of the irradiated light.

3. The testing device of claim 2, further comprising the or more lateral flow assays, preferably wherein the lateral flow assay is affixed to the testing device at the receptacle, wherein preferably the lateral flow assay comprises a sample pad, wicking pad, a test area and a control area, wherein the test area is arranged to change colour when the target substance is present in the liquid sample, and wherein the control area is arranged to change colour based on the status of the biochemical test.

4. The testing device of any of the preceding claim, wherein the testing device comprises one or more openings for an analyte input, preferably a sample well, for inputting the liquid sample into the testing device and receptacle, arranged to bring the liquid sample into contact with the lateral flow test,
wherein preferably a circuit board comprising the one or more sensor systems also has an opening for analyte input to allow specimen to be provided on a side of the lateral flow test facing the sensor systems of the circuit board.

5. The testing device of any one of the preceding claims, comprising a first and second sensor system, wherein the first and second sensor system are simultaneously arranged to emit light towards and measure the optical property from two distinct areas of the biochemical test that change colour, preferably a test area and a control area of a lateral flow test,
wherein preferably the testing device comprises a third sensor system, wherein the third sensor system is arranged to emit light towards and measure the optical property from a reference area on the biochemical test which does not change colour, so as to perform a reference measurement.

6. The testing device of any of the preceding claims, wherein the testing device comprises a housing, wherein the microcontroller and the one or more sensor systems are arranged on a circuit board, preferably an elongated circuit board, wherein preferably a first sensor system is arranged on first long side of the circuit board and a second system is arranged on the opposite, second long side of the circuit board, wherein the housing comprises two receptacles, the circuit board configured sandwiched between the receptacles.

7. The testing device of any of the preceding claims, comprising a transmitter for transmitting the processed data of the one or more sensor systems, the transmitter preferably connected to a microprocessor that is connected to the one or more sensor systems, preferably all mounted on a circuit board
wherein preferably the transmitter is arranged as a transponder with respect to an initiator device that provides an electromagnetic field which the transponder modulates,
wherein preferably the testing device draws all its operating power from the electromagnetic field provided by the initiator device
wherein preferably the transmitter is arranged to use the Near-Field Communication, NFC, communication protocol.

8. The testing device of any of the previous claims, wherein a circuit board comprising one or more sensor systems comprises a unique code, e.g. in a memory, wherein the unique code can be read out via a transmitter, and wherein preferably the transmitter is arranged to provide data based on detection by the sensor systems in combination with the unique code.

9. The testing device of any of the previous claims, wherein the housing has an opening for specimen insertion, the opening aligned with an area of the receptacle arranged to provide a test area of the lateral flow test, wherein preferably the opening is provided with a removable protection, wherein preferably the testing device is arranged to detect removal of the removable protection and more preferably is arranged to process the removal detection result in the output data.

10. Kit of parts comprising a testing device and one or more lateral flow tests arranged to detect the presence of a target substance in a liquid sample, wherein
the testing device has one or more receptacles for receiving one or more lateral flow tests, one or more sensor systems for detecting results provided by the lateral flow test, a microcontroller connected to the one or more sensor systems for processing data from the one or more sensor systems;
the lateral flow test has at least a sample pad, a test area and a control area, wherein the test area and control area will provide a test result detectable by the sensor system
wherein the testing device comprises a housing with an opening aligned with a receptacle, the opening arranged for insertion and the receptacle arranged positioning of at least a part of the lateral flow test having the test area and control area,
wherein the lateral flow test is dimensioned such that the inserted lateral flow test extends from the opening with the sample pad.

11. A method of electronically reading a lateral flow test arranged to detect the presence of a target substance in a liquid sample, the method comprising:
obtaining a biochemical test that has been performed on a liquid sample;
irradiating with visible light an area of the lateral flow test that changes colour, wherein the visible light is of a different colour than the colour to which the area can change;
measuring an optical property of the area that is irradiated to obtain measurement data;
digitally processing the measurement data so that the lateral flow test can be electronically read.

12. The method of claim 11, wherein the lateral flow test is a lateral flow assay, comprising a sample pad, wicking pad, a test area and a control area, wherein the test area is arranged to change colour when the target substance is present in the liquid sample, and wherein the control area is arranged to change colour based on the status of the biochemical test.

13. The method of claim 11 or 12, wherein light is simultaneously emitted towards two distinct areas of the biochemical test that change colour, and the optical property from the two distinct areas are simultaneously measured, preferably wherein the two areas are the test area and the control area.

14. The method of anyone of claims 11-13, wherein light is emitted towards a reference area on the lateral flow assay and wherein the optical property is measured from the reference area on the lateral flow assay, which does not change colour, so as to perform a reference measurement.

15. The method of any one of claims 11-14, further comprising transmitting the digitally processed measurement data, preferably wherein the transmitting is performed using a transmitter which is arranged as a transponder with respect to an initiator device that provides an electromagnetic field which the transponder modulates, preferably wherein the testing device draws all its operating power from the electromagnetic field provided by the initiator device, preferably wherein the transmitter is arranged to use the Near-Field Communication, NFC, communication protocol.
